# EUROPEAN PATENT SPECIFICATION

(11) **EP 3 743 266 B1**
(45) Date of publication and mention of the grant of the patent: **19.04.2023**
(21) Application number: 19701832.8
(22) Date of filing: 24.01.2019
(51) Int. Cl.: B29C 64/393, B33Y 50/02, G02B 21/34, B33Y 10/00, B29C 64/245, B41J 3/407, C12M 1/34, B29K 105/00, B29L 31/00, C12M 1/26, B33Y 30/00

(54) **3D BIOPRINTERS WITH CELL CULTURE MONITORING MEANS**
3D-BIODRUCKER MIT MITTELN ZUR ÜBERWACHUNG VON ZELLKULTUREN
BIO-IMPRIMANTES 3D COMPORTANT DES MOYENS DE SURVEILLANCE DE CULTURE CELLULAIRE

(30) Priority: 24.01.2018 SE 1850073; 24.01.2018 US 201862621091 P
(43) Date of publication of application: 02.12.2020
(73) Proprietor: CELLINK Bioprinting AB, 412 77 Göteborg (SE)
(72) Inventor: MARTINEZ, Hector, 413 09 Göterborg (SE); GATENHOLM, Erik, 411 33 Göteborg (SE)
(74) Representative: Zacco Sweden AB
(86) International application number: PCT/EP2019/051769
(87) International publication number: WO 2019/145433

(56) References cited:
- WO-A1-2015/054577
- Brad Olson ET AL: "Hemocytometer Counting Alternative", Genetic Engineering & Biotechnology News, 1 August 2012 (2012-08-01), XP055583226, Retrieved from the Internet: URL:https://www.genengnews.com/magazine/18 6/hemocytometer-counting-alternative/2/?kw rd=clinical%20reference [retrieved on 2019-04-25]

## Description

### TECHNICAL FIELD

The present disclosure relates to a 3D bioprinter.

### BACKGROUND

Bioprinting, an additive manufacturing technology, has gained due attention for its ability to spatially control the placement of cells, biomaterials and biological molecules. Consequently, it offers endless possibilities to the future of tissue and organ regeneration, basic research and drug screening.

The 3D bioprinter is able to dispense materials while moving in X, Y, and Z directions. This enables the engineering of complex structures from the bottom up. Moreover, this technology allows the biofabrication of biomimetic-shaped 3D structures unique to the target tissue or organ, since it can be combined with CAD/CAM technology using patients' medical images. In a pre-bioprinting process, a model for use by the 3D bioprinter is created. Further, materials that will be used are chosen. Common technologies used for bioprinting are computed tomography (CT) and magnetic resonance imaging (MRI). To print with a layer-by-layer approach, tomographic reconstruction can be done on the images. The now-2D images can then be used by the printer. A 3D bioprinting system is known from WO 2015/054577 A1.

The inventors of the present invention has identified a need for an improved bioprinter system, which provides for improved results in bioprinting.

### SUMMARY

The invention is defined by claims 1 and 16.

In accordance with the present invention a bioprinter system comprises a printbed, at least one of an exchangeable and/or fixed toolhead and a cell culture monitor device. The monitor device comprises a camera arranged to provide images of a cell culture or construct at the printbed. The system further comprises a processing element arranged to monitor cell status in the cell culture or construct at the printbed based on the provided images.

Further, a method is disclosed for monitoring a cell culture or construct at a bioprinter printbed. The method comprises providing images of the cell culture or construct, and monitoring cell status at the printbed based on the provided images.

### SHORT DESCRIPTION OF THE DRAWINGS

Figure 1 shows a bioprinter system.
Figure 2 illustrates one aspect of the present disclosure.
Figure 3 illustrates other aspects of the present disclosure.
Figure 4 illustrates further aspects of the present disclosure.

### DETAILED DESCRIPTION

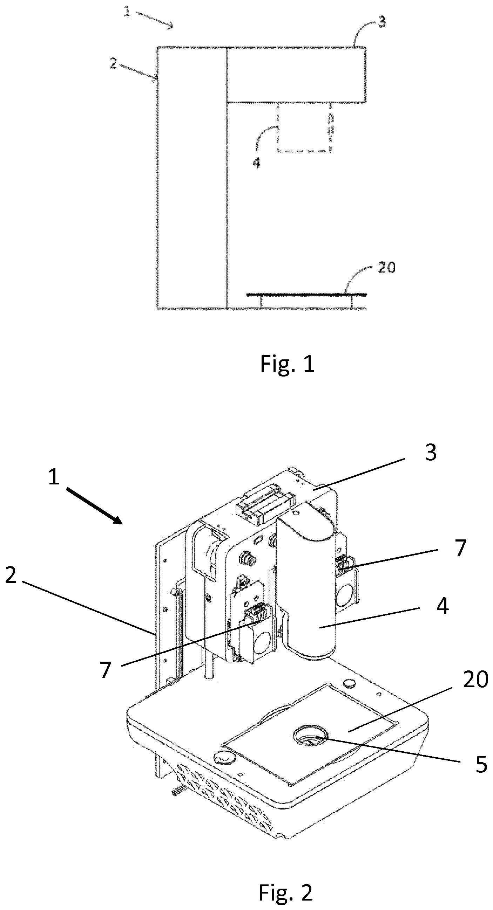

Fig 1 discloses a schematic view of an example of a 3D bioprinter 1 as disclosed herein. The 3D bioprinter can be used for manufacture of three-dimensional engineered biological tissues. The 3D bioprinter 1 may be used in printing constructs that are suitable for use in any of the applications chosen from: implants in the animal or human body, such as repairing or replacing tissue, topical applications, cosmetic applications and drug test applications, as well as any other suitable applications. Printed constructs may include, but are not limited to, tissue-like constructs, constructs with or without living cells, cell cultures, tissue implants and three-dimensional matrices.

The 3D bioprinter 1 may comprise a base unit 2. The base unit 2 may comprise a support 3 adapted for mounting of at least one toolhead 4. In some aspects the support 3 is adapted to accommodate a plurality of toolheads 4.

Figures 2, 3 and 4 illustrate a number of different aspects of a 3D bioprinter as disclosed herein. Notably, even though specific combinations are shown in the figures and described herein, it is understood that specific features may be combined in further suitable manners.

In one aspect, a support 3 is adapted for mounting of at least one exchangeable toolhead 4. Thus, as seen in Figures 2-4, the support may comprise one or more attachment elements 7, adapted for mounting a toolhead 4. In the figures, one single toolhead 4 is shown as mounted on the support 3, and it is understood that such toolhead may be either integrated or exchangeable. Further, the term "integrated" may mean either permanently integrated or mounted in a non-exchangeable manner.

If one or more exchangeable toolhead(s) are used, such toolheads may then be dismounted and displaced with other toolhead(s). In one example, the support 3 is adapted for mounting of at least one non-exchangeable toolhead. In another example, the support may be adapted for mounting of at least one exchangeable toolhead 4 and at least one non-exchangeable toolhead 4 (not illustrated).

In the example where the support 3 is adapted for mounting of at least one exchangeable toolhead, the 3D bioprinter becomes very flexible and efficiency in operation of the 3D bioprinter can be increased. For example, the user can prepare the toolhead outside the 3D bioprinter, wherein the preparation may for example involve loading material and/or selecting needle, and/or calibrating or setting up a sensor or other device. Thereafter the currently used toolhead can be substituted with the new one.

Thereby the bioprinter does not need to be stopped for time consuming activities such as loading of material and/or changing needle, calibration or setting up. Instead, a short stop can be made to substitute the currently used toolhead with the prepared one. Alternatively, the currently used toolhead can be exchanged when printing. For example, if the support 3 is adapted to mount a plurality of toolheads, the exchange can easily be done while printing.

The toolhead can be inserted or removed by a user with or without use of a tool. Alternatively, the toolhead can be automatically inserted and/or removed by the 3D bioprinter itself.

The connection of a toolhead can require one or a plurality of steps. It may for example involve inserting a nut and/or connecting a cable and/or connecting gas supply.

The at least one toolhead 4 may include at least one actuating toolhead and/or at least one sensor toolhead. An actuating toolhead may also be referred to as a printhead, especially in those cases where it adapted for bioprinting. Thus, the expression "toolhead/ is meant to encompass all types of tools used in a bioprinter. Examples of actuating toolheads include pneumatic extrusion toolheads, syringe pump toolheads, inkjet toolheads, high temperature extrusion toolheads, toolheads for removal of material and/or curing toolheads. Toolheads for removal of material may comprise a knife and/or a laser toolhead and/ or a milling toolhead and/or a drilling toolhead and/or a needle and/or tip adapted for suction and/or addition of material. The curing toolheads may comprise a curing UV toolhead and/or a visible light curing toolhead and/or a laser light curing toolhead. Other examples of actuating toolheads include exhaust gas toolheads for exhaust of gas such as air.

The actuating toolhead may be provided with sensor(s). Examples of sensing toolheads include camera toolheads, probing toolheads and/or 3D scanning toolheads.

A bioprinter system according to the present disclosure may be arranged in several different manners. A number of different combinations are disclosed herein, all comprising a cell culture monitor device within or arranged in connection to a bioprinter. The cell culture monitor may be any type of monitor capable of monitoring cell status, such as cell growth, cell viability, histology, cell distribution, cell density, number of cells and/or types of cells. Further, the term "cell culture" in intended to encompass all types of environments wherein living cells may be sustained and cultured. Examples include, but are not limited to, standard cell culture vessels such as petri dishes, multi-well culture vessels, microscope slides, cell culture flasks and tubes etc., but also within a bioprinter construct, such as within a matrix of supporting material. The monitor device may further be adapted to sense, map or detect e.g. cell or tissue markers, such as for instance fluorescently tagged markers, enzymatically tagged markers and/or immunologically tagged markers.

A cell culture monitor device as disclosed is preferably intended for monitoring of the cells in a bioprinted construct or cell culture, during the bioprinting process, and/or after bioprinting in the bioprinter system. Further, it is also possible that the cell culture monitor device may be used to monitor cells in a construct, cell culture or tissue to be used in the bioprinter before and/or during bioprinting.

The cell culture monitor may be fully or partially automated in its function. Further, the cell culture monitor may comprise a cell counter.

A cell culture monitor 5 may comprise a camera of any suitable type, such as a standard photography camera, a video camera, an infrared camera, a camera adapted to detect specific ranges of light, a microscope or other imaging device. The term "camera" is herein intended to comprise, be part of or be any type of optical imaging system capable of monitoring cell status by obtaining images. Such images may be live, still or video images.

In one aspect, as schematically illustrated in Figure 2, a bioprinter system 1 comprises a cell culture monitor device 5 that fits in the printbed 20 of a bioprinter. The cell culture monitor device 5 is in this example an exchangeable device arranged to be received by the printbed 20 or mounted in connection to the printbed 20. It may be provided in a dedicated mounting space, such as a hole or recess in the printbed 20, preferably such that the device is essentially flush with the top surface of the printbed when arranged in the printbed. As an alternative, not illustrated, the cell culture monitor device 5 may be provided on top of the printbed 20, preferably having a flat and wide enough top surface for a petri dish or other type of cell culture container to be placed on top of the monitor device 5.

Once detached from the bioprinter, the cell culture monitor device 5 can preferably also operate inside any standard cell culture incubator to allow the user to monitor the cells and evaluate the cells optimum culture conditions. The cell culture monitor device 5 comprises a camera arranged to provide live, video or still images of a cell culture or construct comprising cells, when placed on top of, or above, the monitor device 5. In one aspect, the camera may provide direct live images. In another aspect, the camera may provide still images. These images may be provided in response to a command by a user, or at one or several specified time points , such that the cell culture may be monitored over time. In a third aspect, the camera may provide video images.

Depending on the type of camera used, as well as the ambient light provided around the printbed, an appropriate light source 6 may be provided at a suitable location, such as on the toolhead 4 or support 3, such that light is supplied to the location where the cell culture or construct is arranged (not shown). Such a light source may further be arranged as an attachment to the monitor device 5 itself (not shown), such that when the monitor device 5 is detached from the bioprinter, the light source will remain connected to the device 5 and continue to illuminate the cell culture.

The bioprinter comprises a processing element arranged to monitor cell status at the printbed 20. Cell status may any parameter used to monitor cells in culture or tissue, such as, but not limited to one of or any combination of: cell growth, cell count, cell viability, cell shape, cell size, cell differentiation, cell density, cell distribution, confluency of cells, cell type or infection by other cell types. The processing element uses information acquired from the obtained images to monitor cell status. It may also use information based on the spatial relation between the camera and the monitored cell culture or construct.

Further, the monitor device 5 may be integrated with analysis software adapted to monitor the cells over a prolonged and continuous period of time, preferably automatically. For example, the monitor device 5 and analysis software may be arranged to obtain time lapse videos of the cell status. For instance, the system could also analyze the confluency over the cell culture period and the processing element may preferably provide warnings to the user, for example by sending a text message and/or email, when something goes wrong with cell culture status or if there is an indication of contamination or infection in the cell culture. In other words, the bioprinter system may be arranged to provide an alert signal when the cell culture status reaches at least one predetermined criteria.

The bioprinter system may be adapted such that the camera is arranged to perform a scanning movement to scan the cell culture or construct at the printbed 20. In addition, a bioprinter system with a cell culture monitor device 5 may be designed to scan and analyze wellplates as well as any cell culture tissue flask, vessel and surface, such as a microfluidic device. Such a system preferably moves the camera instead of moving the samples or construct. This makes the system very fast and accurate in scanning and analysis. A bioprinter system with a camera arranged for scanning and movement preferably comprises a control element arranged to obtain control signals for controlling the scanning movement. The scanning movement of the camera may be controlled manually by a user and/or automatically by the system.

The scanning movement is preferably performed by an actuator, and/or motor, and controlled by the control element.

The system could also include an automated cell counter by including any disposable and/or reusable hemocytometer. In such a setup, the system could perform a single or multiple measurements to evaluate important parameters such as cell viability, concentration, shape, size, among others. The automatic cell counter feature allows a user to evaluate the cell numbers and viability before a bioprinting experiment. These two factors are extremely important to control and evaluate before embedding the cells in the bioink, in order to guarantee a desired final cell concentration in the printed tissue. The system may also provide valuable information of cell status during and after a bioprinting process.

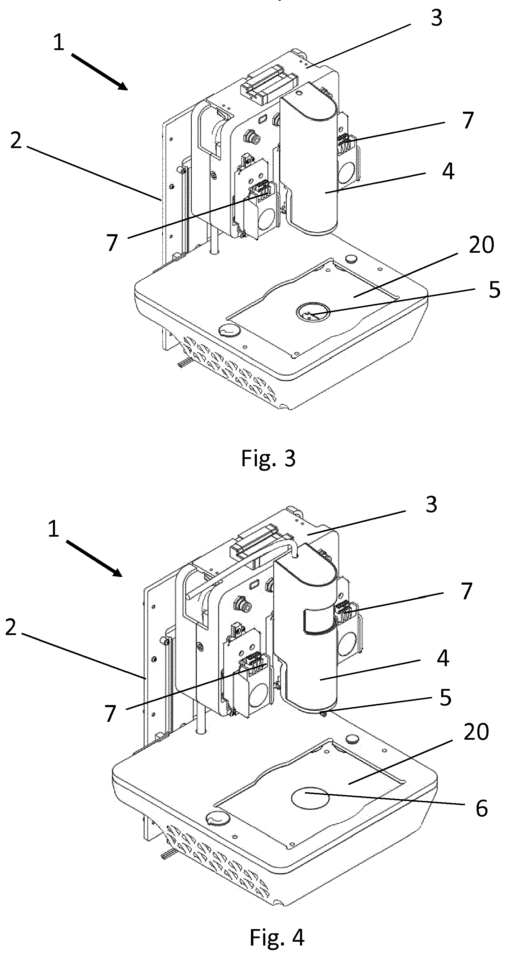

As illustrated in Figure 3, the cell culture monitor device 5 is integrated in the printbed of a bioprinter. It may be located anywhere in the printbed; center, side, back, front, as well as on the side of the printbed, e.g. as a plugin device. It may be arranged under, on top of or in the printbed, preferably essentially flush with the top surface of the printbed. The user may use this device to monitor the cells over the cell culture period and evaluate their status, such as cell growth, cell count or cell viability.

All other aspects of the bioprinter 1 and cell culture monitor device 5 shown in Figure 3 may be as described above in connection with Figure 2. Thus, the cell culture monitor device 5 comprises a camera arranged to provide live, video or still images of a cell culture, or construct comprising cells, arranged on the printbed 20 and monitor device 5.

Further, the monitor device 5 may be integrated with analysis software to automatically monitor the cells over a prolonged and continuous period of time. In addition, the device may be adapted to scan and analyze different types of cell culture surfaces, and the device may be adapted to move the camera instead of moving the cell culture when scanning.

The device may be adapted to be used as a cell counter. The automatic cell counter feature allows a user to evaluate the cell numbers and viability before a bioprinting experiment. These two factors are extremely important to control and evaluate before embedding the cells in the bioink in order to guarantee a desired final cell concentration in the printed tissue.

In a third aspect, as illustrated in Figure 4, the bioprinter system 1 may comprise a cell culture monitor device 5 that is mounted above the printbed. Such a monitor device 5 may be e.g. integrated in, part of, or attached to an exchangeable or fixed toolhead 4 of a bioprinter.

In the case of a monitor device 5 being part of or comprised by an exchangeable toolhead 4, once detached from the bioprinter, such a cell culture monitor device 5 may be adapted to be mounted on a stand-alone mount or holder to be able to operate inside any standard cell culture incubator, for instance to allow the user to monitor cells and evaluate the cells optimum culture conditions. The monitor device 5 may also be adapted to be used without a holder inside any standard cell culture incubator.

In one aspect, the bioprinter may comprise an appropriate light source 6 at a suitable location, such as in or near the printbed 20, such that light is supplied to the location where the cell culture or construct is arranged. In the example of Figure 4, a light source 6 is mounted within the printbed 20. Such a light source may further be arranged as an attachment to the monitor device 5 itself (not shown), such that when the monitor device 5 is detached from the bioprinter, the light source will remain connected to the device 5 and continue to illuminate the cell culture or construct.

All other aspects of a bioprinter and cell culture monitor device 5 as shown in Figure 4 may be as described above in connection with Figure 2 and 3. Thus, the cell culture monitor device 5 comprises a camera arranged to provide live, video or still images of a cell culture, or construct comprising cells, arranged on the printbed 20.

Further, the monitor device 5 may be integrated with analysis software to automatically monitor the cells over a prolonged and continuous period of time. Further, the device may be adapted to scan and analyze different types of cell culture surfaces.

In the bioprinter shown in Figure 4, since the monitor device 5 may be attached to or integrated with a toolhead or toolhead holder, it may further be adapted to move the camera, for instance in in X and Y axes, instead of moving the cell culture or construct. This makes the system very fast and accurate in scanning and analysis.

The monitor device 5 may be adapted to be used as a cell counter. The automatic cell counter feature allows a user to evaluate the cell numbers and viability before bioprinting experiments. These two factors are extremely important to control and evaluate before embedding the cells in the bioink in order to guarantee a desired final cell concentration in the printed tissue.

The present invention is not limited to the above-described preferred embodiments. Various alternatives, modifications and equivalents may be used. Therefore, the above embodiments should not be taken as limiting the scope of the invention, which is defined by the appending claims.

## Claims

1. A bioprinter system (1) comprising
a printbed (20),
at least one of an exchangeable and/or fixed toolhead (4),
a cell culture monitor device (5), the device (5) comprising a camera arranged to provide images of a cell culture or construct at the printbed (20), and
a processing element arranged to monitor cell status in the cell culture at the printbed or to monitor the construct at the printbed (20) based on the provided images, **characterized in that** the monitor device (5) is integrated with the printbed (20) of the bioprinter.

2. The bioprinter system (1) according to claim 1, further comprising a light source (6) arranged to illuminate the cell culture or construct at the printbed (20).

3. The bioprinter system (1) according to any of the preceding claims, wherein the camera provides still images.

4. The bioprinter system (1) according to any of the preceding claims, wherein the camera provides video images.

5. The bioprinter system (1) according to any of the preceding claims, wherein the camera provides images at specified time points.

6. The bioprinter system (1) according to any of the preceding claims, further comprising a hemocytometer arranged to perform a cell count in the cell culture or construct.

7. The bioprinter system (1) according to any of the preceding claims, wherein the processing element is arranged to determine a cell viability and/or concentration based on the acquired images and/or cell count.

8. The bioprinter system (1) according to any of the preceding claims, wherein the processing element is arranged to determine a cell size and/or cell shape of the cell culture or construct based on the acquired images and/or the cell count.

9. The bioprinter system (1) according to any of the preceding claims, wherein the processing element is arranged to analyse a confluency over a cell culture period based on the acquired images and/or the cell count.

10. The bioprinter system (1) according to any of the preceding claims, wherein the processing element is arranged to provide an alert signal when the cell culture status reaches at least one predetermined criteria.

11. The bioprinter system (1) according to claim 10, further comprising a communication element arranged to provide a warning to a user upon receipt of the alert signal, wherein the warning for example is a text message such as an e-mail message and/or an audio signal and/or a visual signal or display.

12. The bioprinter system (1) according to any preceding claim, wherein the camera is arranged to perform a scanning movement to scan the cell culture or construct at the printbed (20).

13. The bioprinter system (1) according to claim 12, further comprising a control element arranged to obtain control signals for control of the scanning movement.

14. The bioprinter system (1) according to claim 13, further comprising an actuator or motor arranged to perform the scanning movement, said actuator or motor being controlled by the control element.

15. The bioprinter system (1) according to any of claims 12-14, wherein the processing element is arranged to monitor cell status at the printbed (20) based also on information related to a spatial relation between the camera and the cell culture or construct, for example based on the control signal.

16. A method for monitoring a cell culture or construct at a bioprinter printbed (20), the method comprising:
providing images of the cell culture or construct by using a monitor device (5) being integrated with the printbed (20) of the bioprinter, and
monitoring cell status at the printbed (20) based on the provided images.

17. The method according to claim 16, wherein the monitoring step comprises analysing a confluency over a cell culture period.

18. The method according to claim 16 or 17, wherein monitoring comprises performing a cell count of the cell culture or construct and determining a cell viability and/or cell concentration based on the cell count.

19. The method according to any of the claims 16-18, wherein the monitoring comprises determining a cell viability of the cell culture or construct based on the acquired images and/or cell count.

20. The method according to any of the claims 16-19, wherein monitoring comprises the determining a cell size and/or cell shape of the cell culture or construct based on the acquired images and/or the cell count.

21. The method according to any of the claims 16-20, further comprising a step of providing an alert signal when the cell culture status reaches at least one predetermined criteria.

22. The method according to any of claims 16-21, further comprising providing a warning to a user upon receipt of the alert signal, wherein the warning for example is a text message such as an e-mail message and/or an audio signal and/or a visual signal or display.

23. The method according to any of claims 16-22, wherein the providing of images comprises performing a scanning movement with a camera to scan the cell culture or construct at the printbed (20).

24. Software for monitoring a cell culture or construct at a bioprinter printbed (20) arranged in a bioprinter system according to any of claims 1 to 15 to perform the method according to any of the claims 16-23.

25. Computer program comprising computer program code arranged in a bioprinter system according to any of claims 1 to 15 to when executed perform the method for monitoring a cell culture or construct at a bioprinter printbed (20) according to any of the claims 16-23.

## Patentansprüche

1. Biodruckersystem (1), umfassend
eine Druckplatte (20),
mindestens einen von einem auswechselbaren und/oder einem feststehenden Werkzeugkopf (4),
eine Vorrichtung (5) zur Überwachung von Zellkulturen, wobei die Vorrichtung (5) eine Kamera umfasst, die dazu eingerichtet ist, Bilder einer Zellkultur oder eines Gebildes an der Druckplatte (20) bereitzustellen, und
ein Verarbeitungselement, das dazu eingerichtet ist, den Zellenstatus in der Zellkultur an der Druckplatte zu überwachen, oder das Gebilde an der Druckplatte (20) basierend auf den bereitgestellten Bildern zu überwachen,
**dadurch gekennzeichnet, dass** die Überwachungsvorrichtung (5) mit der Druckplatte (20) des Biodruckers integriert ist.

2. Biodruckersystem (1) nach Anspruch 1, ferner umfassend eine Lichtquelle (6), die dazu eingerichtet ist, die Zellkultur oder das Gebilde an der Druckplatte (20) zu beleuchten.

3. Biodruckersystem (1) nach einem der vorhergehenden Ansprüche, wobei die Kamera Standbilder bereitstellt.

4. Biodruckersystem (1) nach einem der vorhergehenden Ansprüche, wobei die Kamera Videobilder bereitstellt.

5. Biodruckersystem (1) nach einem der vorhergehenden Ansprüche, wobei die Kamera Bilder zu vorgegebenen Zeitpunkten bereitstellt.

6. Biodruckersystem (1) nach einem der vorhergehenden Ansprüche, ferner umfassend ein Hämozytometer, das dazu eingerichtet ist, eine Zellzählung in der Zellkultur oder dem Gebilde vorzunehmen.

7. Biodruckersystem (1) nach einem der vorhergehenden Ansprüche, wobei das Verarbeitungselement dazu eingerichtet ist, eine Zellviabilität und/oder Konzentration basierend auf den erfassten Bildern und/oder der Zellzählung zu bestimmen.

8. Biodruckersystem (1) nach einem der vorhergehenden Ansprüche, wobei das Verarbeitungselement dazu eingerichtet ist, eine Zellengröße und/oder Zellenform der Zellkultur oder des Gebildes basierend auf den erfassten Bildern und/oder der Zellzählung zu bestimmen.

9. Biodruckersystem (1) nach einem der vorhergehenden Ansprüche, wobei das Verarbeitungselement dazu eingerichtet ist, eine Konfluenz über einen Zellkulturzeitraum basierend auf den erfassten Bildern und/oder der Zellzählung zu analysieren.

10. Biodruckersystem (1) nach einem der vorhergehenden Ansprüche, wobei das Verarbeitungselement dazu eingerichtet ist, ein Alarmsignal bereitzustellen, wenn der Zellkulturstatus mindestens ein vorbestimmtes Kriterium erreicht.

11. Biodruckersystem (1) nach Anspruch 10, ferner umfassend ein Kommunikationselement, das dazu eingerichtet ist, einem Benutzer eine Warnung bereitzustellen, wenn das Alarmsignal empfangen wird, wobei die Warnung beispielsweise eine Textnachricht, wie etwa eine E-Mail-Nachricht, und/oder ein Audiosignal und/oder ein visuelles Signal oder eine Anzeige ist.

12. Biodruckersystem (1) nach einem der vorhergehenden Ansprüche, wobei die Kamera dazu eingerichtet ist, eine Abtastbewegung auszuführen, um die Zellkultur oder das Gebilde an der Druckplatte (20) abzutasten.

13. Biodruckersystem (1) nach Anspruch 12, ferner umfassend ein Steuerelement, das dazu eingerichtet ist, Steuersignale zum Steuern der Abtastbewegung zu erzielen.

14. Biodruckersystem (1) nach Anspruch 13, ferner umfassend ein Stellglied oder einen Motor, das bzw. der dazu eingerichtet ist, die Abtastbewegung auszuführen, wobei das Stellglied oder der Motor von dem Steuerelement gesteuert wird.

15. Biodruckersystem (1) nach einem der Ansprüche 12 bis 14, wobei das Verarbeitungselement dazu eingerichtet ist, den Zellstatus an der Druckplatte (20) basierend auch auf Informationen bezüglich einer räumlichen Beziehung zwischen der Kamera und der Zellkultur oder dem Gebilde, beispielsweise basierend auf dem Steuersignal, zu überwachen.

16. Verfahren zum Überwachen einer Zellkultur oder eines Gebildes an einer Biodrucker-Druckplatte (20), wobei das Verfahren umfasst:
Bereitstellen von Bildern der Zellkultur oder des Gebildes unter Verwendung einer Überwachungsvorrichtung (5), die mit der Druckplatte (20) des Biodruckers integriert ist, und
Überwachen des Zellstatus an der Druckplatte (20) basierend auf den bereitgestellten Bildern.

17. Verfahren nach Anspruch 16, wobei der Überwachungsschritt das Analysieren einer Konfluenz über einen Zellkulturzeitraum umfasst.

18. Verfahren nach Anspruch 16 oder 17, wobei das Überwachen das Ausführen einer Zellzählung der Zellkultur oder des Gebildes und das Bestimmen einer Zellviabilität und/oder einer Zellkonzentration basierend auf der Zellzählung umfasst.

19. Verfahren nach einem der Ansprüche 16 bis 18, wobei das Überwachen das Bestimmen einer Zellviabilität der Zellkultur oder des Gebildes basierend auf den erfassten Bildern und/oder der Zellzählung umfasst.

20. Verfahren nach einem der Ansprüche 16 bis 19, wobei das Überwachen das Bestimmen einer Zellengröße und/oder einer Zellenform der Zellkultur oder des Gebildes basierend auf den erfassten Bildern und/oder der Zellzählung umfasst.

21. Verfahren nach einem der Ansprüche 16 bis 20, ferner umfassend einen Schritt des Bereitstellens eines Alarmsignals, wenn der Zellkulturstatus mindestens ein vorbestimmtes Kriterium erreicht.

22. Verfahren nach einem der Ansprüche 16 bis 21, ferner umfassend das Bereitstellen einer Warnung für einen Benutzer, wenn das Alarmsignal empfangen wird, wobei die Warnung beispielsweise eine Textnachricht, wie etwa eine E-Mail-Nachricht, und/oder ein Audiosignal und/oder ein visuelles Signal oder eine Anzeige ist.

23. Verfahren nach einem der Ansprüche 16 bis 22, wobei das Bereitstellen von Bildern das Ausführen einer Abtastbewegung mit einer Kamera umfasst, um die Zellkultur oder das Gebilde an der Druckplatte (20) abzutasten.

24. Software zum Überwachen einer Zellkultur oder eines Gebildes an einer Biodrucker-Druckplatte (20), die in einem Biodruckersystem nach einem der Ansprüche 1 bis 15 eingerichtet ist, um das Verfahren nach einem der Ansprüche 16 bis 23 auszuführen.

25. Computerprogramm, das Computerprogrammcode umfasst, der in einem Biodruckersystem nach einem der Ansprüche 1 bis 15 dazu eingerichtet ist, wenn er durchgeführt wird, das Verfahren zum Überwachen einer Zellkultur oder eines Gebilde an einer Biodrucker-Druckplatte (20) nach einem der Ansprüche 16 bis 23 auszuführen.

## Revendications

1. Système de bio-imprimante (1) comprenant
un lit d'impression (20),
au moins l'une d'une tête d'outil interchangeable et / ou fixe (4),
un dispositif de surveillance de culture cellulaire (5), le dispositif (5) comprenant une caméra agencée pour fournir des images d'une culture ou d'une construction cellulaire au niveau du lit d'impression (20), et
un élément de traitement agencé pour surveiller l'état des cellules dans la culture cellulaire au niveau du lit d'impression ou surveiller la construction au niveau du lit d'impression (20) sur la base des images fournies,
**caractérisé en ce que** le dispositif de surveillance (5) est intégré au lit d'impression (20) de la bio-imprimante.

2. Système de bio-imprimante (1) selon la revendication 1, comprenant en outre une source de lumière (6) agencée pour éclairer la culture ou construction cellulaire au niveau du lit d'impression (20).

3. Système de bio-imprimante (1) selon l'une quelconque des revendications précédentes, dans lequel la caméra fournit des images fixes.

4. Système de bio-imprimante (1) selon l'une quelconque des revendications précédentes, dans lequel la caméra fournit des images vidéo.

5. Système de bio-imprimante (1) selon l'une quelconque des revendications précédentes, dans lequel la caméra fournit des images à des instants spécifiés.

6. Système de bio-imprimante (1) selon l'une quelconque des revendications précédentes, comprenant en outre un hémocytomètre agencé pour effectuer un comptage cellulaire dans la culture ou construction cellulaire.

7. Système de bio-imprimante (1) selon l'une quelconque des revendications précédentes, dans lequel l'élément de traitement est agencé pour déterminer une viabilité et / ou une concentration cellulaire sur la base des images acquises et / ou du comptage cellulaire.

8. Système de bio-imprimante (1) selon l'une quelconque des revendications précédentes, dans lequel l'élément de traitement est agencé pour déterminer une taille de cellule et / ou une forme de cellule de la culture cellulaire ou de la construction cellulaire sur la base des images acquises et / ou du comptage cellulaire.

9. Système de bio-imprimante (1) selon l'une quelconque des revendications précédentes, dans lequel l'élément de traitement est agencé pour analyser une confluence sur une période de culture cellulaire sur la base des images acquises et / ou du comptage cellulaire.

10. Système de bio-imprimante (1) selon l'une quelconque des revendications précédentes, dans lequel l'élément de traitement est agencé pour fournir un signal d'alerte lorsque l'état de la culture cellulaire atteint au moins un critère prédéterminé.

11. Système de bio-imprimante (1) selon la revendication 10, comprenant en outre un élément de communication agencé pour fournir un avertissement à un utilisateur lors de la réception du signal d'alerte, dans lequel l'avertissement est par exemple un message textuel tel qu'un message électronique et / ou un signal sonore et / ou un signal visuel ou un affichage.

12. Système de bio-imprimante (1) selon l'une quelconque des revendications précédentes, dans lequel la caméra est agencée pour effectuer un mouvement de balayage pour balayer la culture ou construction cellulaire au niveau du lit d'impression (20).

13. Système de bio-imprimante (1) selon la revendication 12, comprenant en outre un élément de commande agencé pour obtenir des signaux de commande pour la commande du mouvement de balayage.

14. Système de bio-imprimante (1) selon la revendication 13, comprenant en outre un actionneur ou moteur agencé pour effectuer le mouvement de balayage, ledit actionneur ou moteur étant commandé par l'élément de commande.

15. Système de bio-imprimante (1) selon l'une quelconque des revendications 12 à 14, dans lequel l'élément de traitement est agencé pour surveiller l'état des cellules au niveau du lit d'impression (20) sur la base également d'informations liées à une relation spatiale entre la caméra et la culture ou construction cellulaire, par exemple sur la base du signal de commande.

16. Procédé de surveillance d'une culture ou d'une construction cellulaire au niveau d'un lit d'impression de bio-imprimante (20), le procédé comprenant les étapes consistant à :
fournir des images de la culture ou de la construction cellulaire en utilisant un dispositif de surveillance (5) intégré au lit d'impression (20) de la bio-imprimante, et
surveiller l'état de la cellule au niveau du lit d'impression (20) sur la base des images fournies.

17. Procédé selon la revendication 16, dans lequel l'étape de surveillance comprend l'analyse d'une confluence sur une période de culture cellulaire.

18. Procédé selon la revendication 16 ou 17, dans lequel la surveillance comprend la réalisation d'un comptage cellulaire de la culture ou de la construction cellulaire et la détermination d'une viabilité cellulaire et / ou d'une concentration cellulaire sur la base du comptage cellulaire.

19. Procédé selon l'une quelconque des revendications 16 à 18, dans lequel la surveillance comprend la détermination d'une viabilité cellulaire de la culture ou de la construction cellulaire sur la base des images acquises et / ou du comptage cellulaire.

20. Procédé selon l'une quelconque des revendications 16 à 19, dans lequel la surveillance comprend la détermination d'une taille de cellule et / ou d'une forme de cellule de la culture ou de la construction cellulaire sur la base des images acquises et / ou du comptage cellulaire.

21. Procédé selon l'une quelconque des revendications 16 à 20, comprenant en outre une étape consistant à fournir un signal d'alerte lorsque l'état de la culture cellulaire atteint au moins un critère prédéterminé.

22. Procédé selon l'une quelconque des revendications 16 à 21, comprenant en outre un avertissement à un utilisateur lors de la réception du signal d'alerte, dans lequel l'avertissement est par exemple un message textuel tel qu'un message électronique et / ou un signal sonore et / ou un signal visuel ou un affichage.

23. Procédé selon l'une quelconque des revendications 16 à 22, dans lequel la fourniture d'images comprend l'exécution d'un mouvement de balayage avec une caméra pour balayer la culture ou construction cellulaire au niveau du lit d'impression (20).

24. Logiciel pour surveiller une culture ou construction cellulaire au niveau d'un lit d'impression de bio-imprimante (20) arrangé dans un système de bio-imprimante selon l'une quelconque des revendications 1 à 15 pour exécuter le procédé selon l'une quelconque des revendications 16 à 23.

25. Programme informatique comprenant un code de programme informatique arrangé dans un système de bio-imprimante selon l'une quelconque des revendications 1 à 15 qui, lorsqu'il est exécuté, met en oeuvre le procédé de surveillance d'une culture ou d'une construction cellulaire au niveau d'un lit d'impression de bio-imprimante (20) selon l'une quelconque des revendications 16 à 23.
